# EUROPEAN PATENT SPECIFICATION

(11) **EP 2 891 454 B1**
(45) Date of publication and mention of the grant of the patent: **22.11.2023**
(21) Application number: 13832522.0
(22) Date of filing: 11.07.2013
(51) Int. Cl.: A61B 5/16

(54) **CONCENTRATION RATIO MEASUREMENT DEVICE AND PROGRAM**
VORRICHTUNG UND PROGRAMM ZUR MESSUNG DES KONZENTRATIONSVERHÄLTNISSES
DISPOSITIF ET PROGRAMME DE MESURE DU RAPPORT DE CONCENTRATION

(30) Priority: 31.08.2012 JP 2012191745; 28.03.2013 JP 2013069921
(43) Date of publication of application: 08.07.2015
(73) Proprietor: Panasonic Intellectual Property Management Co., Ltd., Osaka-shi, Osaka 540-6207 (JP)
(72) Inventor: OOBAYASHI, Fumiaki, Osaka 540-6207 (JP); IWAKAWA, Mikio, Osaka 540-6207 (JP); SHIMODA, Hiroshi, Kyoto-shi Kyoto 606-8501 (JP); ISHII, Hirotake, Kyoto-shi Kyoto 606-8501 (JP); MIYAGI, Kazune, Kyoto-shi Kyoto 606-8501 (JP); KUNIMASA, Shutaro, Kyoto-shi Kyoto 606-8501 (JP); SEO, Kyoichi, Kyoto-shi Kyoto 606-8501 (JP); OISHI, Kotaro, Kyoto-shi Kyoto 606-8501 (JP); UCHIYAMA, Kosuke, Kyoto-shi Kyoto 606-8501 (JP)
(74) Representative: Appelt, Christian W.
(86) International application number: PCT/JP2013/004284
(87) International publication number: WO 2014/034008

(56) References cited:
- JP-A- H0 767 862
- JP-A- 2001 120 522
- JP-A- 2001 120 522
- JP-A- 2005 071 250
- JP-A- 2006 158 421
- US-A- 4 770 636
- US-A- 6 066 092
- US-A1- 2002 192 624
- US-A1- 2003 157 469
- US-B1- 7 837 472
- KOTARO OISHI ET AL.: 'Sagyo eno Shuchu ni Chakumoku shita Chiteki Seisansei Hyoka Tool no Teian' THE HUMAN INTERFACE SYMPOSIUM RONBUNSHU 04 September 2012, XP008176123

## Description

### Technical Field

The present invention relates to a concentration ratio measurement apparatus that can objectively measure a concentration ratio of a worker performing a mental work such as a work person in an office, and a program for realizing, to a computer, a function of a main part of the concentration ratio measurement apparatus.

### Background Art

Conventionally, as technology of measuring a concentration of a worker, JP 09-135826 A proposes a technology that makes a test subject trace a standard figure and calculate a concentration ratio with a shift amount between the standard figure and a traced figure. The concentration ratio is calculated as a value obtained by multiplying, by the coefficient, a value calculated from the shift amount between the standard figure and the traced figure. Then, when the trace work is finished, a change, an average value, a standard deviation, a coefficient of variation, a maximum value, a minimum value, and the like of the concentration ratio, which are calculated at constant intervals during the trace work, are calculated. JP 09-135826 A discloses changing difficulty by changing a speed of the trace work when the trace of a standard figure is performed, and presuming a physiology state or a personality characteristic of the test subject by performing the trace work of the standard figure.

JP 2006-87743 A shows a technology that measures a recognition capability of a test subject, a technology that shows, to the test subject, an image generated by degrading an image including a meaningful object, and calculates a capability score with a time period (sensation time period) until the test subject perceives the object. JP 2006-87743 A discloses a technology that calculates beforehand difficulty information at the time of perceiving the image by showing the image to two or more persons, and calculates the capability score of the test subject by using the difficulty information of the image and the sensation time of showing the image to a specific test subject.

In the technology disclosed in JP 09-135826 A, it is possible to calculate change of the concentration ratio of the test subject while a problem called the trace work is performed. However, since being not the mental task load, the technology disclosed in JP 09-135826 A is not suitable for the evaluation of the intellectual productivity. The trace work depends on the hand's athletic ability of the test subject. In this point, the trace work is not suitable for the purpose of evaluating the concentration ratio objectively. The mental work load is used here in the meaning shown in the Japanese industrial Standard "JIS Z8502". The Japanese Industrial Standard "JIS Z8502-1994" is a standard based on international standard of International Organization for Standardization (ISO) "ISO 10075 (Ergonomic principle related to mental workload-General terms and definitions)".

On the other hand, since the capability score disclosed in JP 2006-87743 A is the problem that the meaning of each image is perceived, a mental work load is given to the test subject by problem. However, in the technology disclosed in JP 2006-87743 A, the capability to perceive the meaning of each image is only measured. That is, in the technology disclosed in JP 2006-87743 A, it is hard to evaluate, with the capability score, the concentration ratio when the mental work load is given to the test subject.

US 4,770,636 A discloses a cognometer. A cognometer is a portable electronic device programmed for repeated, rapid, automated assessment and monitoring of memory and concentration. The memory monitor randomly generates data and displays the same in a common format, tests the user's comprehension of such data, tests the immediate recall of such data, and tests the delayed recall of such data. The concentration monitor randomly generates a long multi-digit number and displays the same, tests the user's comprehension of such number, and tests the concentration of the user by requiring the user to enter such number while the number is displayed, but without any position cue.

JP 2001/120522 A provides a processing capability inspection device for inspection of a processing capability for a subject's applied stimulus, which can accurately measure and analyze a temporal change of a response to a stimulus to at least one of visual, auditory, and tactile sensations of the subject. Provided is a processing capability inspection device for inspection.

US 7,837,472 B1 shows a computerized Neuropsychological/ NeuroCognitive and Psychomotor Performance Assessment and Rehabilitation system that is designed for use on handheld computer systems. Components of the system include an executive program, test modules, interpretive and report modules, and supporting utilities. The system provides point-of-use interpretations and result reports. The system is designed for use in clinical settings, occupational medicine, and research. Medical applications include use as a diagnostic, evaluation, and treatment instrument. In industrial settings it can be used as a fitness/readiness for work assessment. The assessment and rehabilitation system also contains modules for use in forensic mental competency, mental and emotional status examinations.

US 2003/0157469 A1 relates to a computer program for generating educational and psychological test items. A computer program and a method for creating non-verbal test items to target a level and a cognitive source of item difficulty for psychological tests comprising a test configuration module for choosing an ability to be tested, an item type, and a general appearance of the items, an item specification module for choosing one of a plurality of blueprints, and an item creation module for creating each item. Each item comprises a stem and a list of response alternatives. Both the stem and the list of response alternatives each comprise a plurality of objects. The blueprints allow a user to create and immediately use the items without having to conduct empirical tryouts. Tests can be administered either as fixed-content paper tests, fixed-content computer tests, or adaptive-content computer tests.

### Summary of Invention

The present invention is defined in claims 1 and 7.

An object of the present invention is to provide a concentration ratio measurement apparatus that can objectively measure a concentration ratio when a mental work load is given. Also, the object of the present invention is to provide a program for realizing, to a computer, a function of a main part of the concentration ratio measurement apparatus.

A concentration ratio measurement apparatus according to the present invention includes: a presentation device, an input device, and an evaluation device. The presentation device is configured to present a objects, a plurality of answer options for selection by the test subject using an input device, wherein each recognition object presented to the test subject is associated to a work problem to be answered by the test subject. The input device is configured to allow the test subject input, for each of the plurality of recognition objects presented by the presentation device, a selection of an answer option out of the plurality of answer options, wherein the selected answer option is an answer to the respective work problem associated with the respective recognition object presented to the test subject. The evaluation device is configured to evaluate a concentration ratio of the test subject on the mental work using at least one of right or wrong of the answer and an answering time from a time when the recognition object is presented on the presentation device to a time when the answer is inputted into the input device, for each of the plurality of recognition objects. The work problem includes: a work of extracting two or more kinds of prescribed cognitive elements included in the recognition object, for each of the plurality of cognition objects; and a work of choosing, from a plurality of options, an option suiting to the two or more prescribed cognitive elements extracted from the recognition object, for each of the plurality of cognition objects, a number of the plurality of options being a number of combinations of selections set to each of the two or more kinds of prescribed cognitive elements. The evaluation device includes a recognition object storage part, a presentation control part, a work memory part, and an evaluating arithmetic part. The recognition object storage part is configured to store the plurality of recognition objects. The presentation control part has: a function of making the presentation device present the plurality of recognition objects stored in the recognition object storage part; and a function of making the presentation device present the plurality of options for each of the plurality of recognition objects. The work memory part is configured to store, as work information, the at least one of the answering time and the right or wrong of the answer for each of the plurality of recognition objects. The evaluating arithmetic part is configured to calculate an evaluation value of the concentration ratio using a statistics value of the work information stored in the work memory part while the plurality of recognition objects are presented on the presentation device. Each of the plurality of recognition objects presented by the presentation device (20) to the test subject has three or more prescribed part is configured to store, as work information, the at least one of the answering time and the right or wrong of the answer for each of the plurality of recognition objects. The evaluating arithmetic part is configured to calculate an evaluation value of the concentration ratio using a statistics value of the work information stored in the work memory part while the plurality of recognition objects are presented on the presentation device.

In the concentration ratio measurement apparatus, the evaluating arithmetic part includes a histogram generating part, an applying part, and a calculation part. The histogram generating part is configured to classify the answering times into a plurality of sections, the histogram generating part being configured to regard, as a time occupancy degree, a ratio of a total of the answering times in a section to a total of the answering time for each of the plurality of sections, the histogram generating part being configured to generate a time occupancy degree histogram expressing a distribution of the time occupancy degree. The applying part is configured to regard the time occupancy degree histogram as a superimposition of a probability density function of a first log normal distribution, which is applied to a first mountain-shaped part with a first peak part in which the time occupancy degree is the largest, and a probability density function of a second log normal distribution, which is applied to a second mountain-shaped part with a second peak part in which the time occupancy degree is the next largest. The calculation part is configured to extract, as a feature amount, an expected value calculated from the probability density function of the first log normal distribution, and calculate, as a concentration time, a product of the feature amount and a total number of the answer, the calculation part being configured to calculate, as the evaluation value, the concentration time to a measurement period that is a total of the answering times.

In the concentration ratio measurement apparatus, the calculation part is preferably configured to calculate, as a non-concentration time in the measurement period, a value obtained by subtracting the concentration time from the measurement period.

In the concentration ratio measurement apparatus, preferably, each of the plurality of recognition objects is three or more-digit number. Each of the two or more kinds of prescribed cognitive elements is a figure of each digit of the number. The selections are number sets including two or more figures, the number sets are divided into three or more kinds of groups. The work is a work of classifying each of the two or more kinds of prescribed cognitive elements into a number set to which each of the two or more kinds of prescribed cognitive elements belongs.

In the concentration ratio measurement apparatus, preferably, each of the plurality of recognition objects is a word presented so that three or more kinds of cognitive elements is extractable as the two or more kinds of prescribed recognition elements.

Preferably, the concentration ratio measurement apparatus further includes an environment sensor and a result storing part. The environment calculated for each of the plurality of recognition objects and the right or wrong of the answer, is used for the evaluation value on the concentration ratio of the test subject, the concentration ratio when the mental work load is given is measured objectively.

### Brief Description of Drawings

Preferable embodiments according to the present invention will be described in more detail. Other features and advantages of the present invention will be better understood with reference to the following detailed description and the attached drawings:
FIG. 1 is a block diagram illustrating a concentration ratio measurement apparatus according to a first embodiment;
FIG. 2A is a drawing illustrating an example of a recognition object according to the first embodiment, and FIG. 2B is a drawing illustrating a display example of an input device according to the first embodiment;
FIG. 3Ais a drawing illustrating another example of the recognition object according to the first embodiment, and FIG. 3B is a drawing illustrating a display example of the input device according to the first embodiment;
FIG. 4 is a drawing illustrating a relation between an elapsed time and an answering time in the concentration ratio measurement apparatus according to the first embodiment;
FIG. 5 is a drawing illustrating an example of a time occupancy degree histogram used for the concentration ratio measurement apparatus according to the first embodiment;
FIG. 6 is a drawing illustrating an example of a histogram used for a concentration ratio measurement apparatus according to a second embodiment; and
FIG. 7 is a block diagram illustrating another configuration example of the concentration ratio measurement apparatus according to the second embodiment.

### Description of Embodiments

In an embodiment described below, a case is assumed, in which a test subject is a worker in an office. The worker in the office mainly performs not a physical work obtaining a work result by a motion of a body but a mental work or an intellectual task that are performed using knowledge, such as a document preparing, an information management, or a classifying work. As for the productivity of a mental work, not only personal capability but the concentration ratio at the time of work influences. The concentration ratio at the time of work is affected by the influence of the various conditions including the environmental elements, such as illumination of working clearance, temperature, humidity, noise, and a bad smell, a recess, for example. Therefore, if the concentration ratio at the time of the work on various conditions is evaluated, it is possible to find out the conditions that improve the concentration ratio, and leading to improvement in the productivity in the mental work is expected through an improvement of this kind of conditions. The test subject whose concentration ratio is measured may be not only the work person in an office but a school, a student in a home, for example.

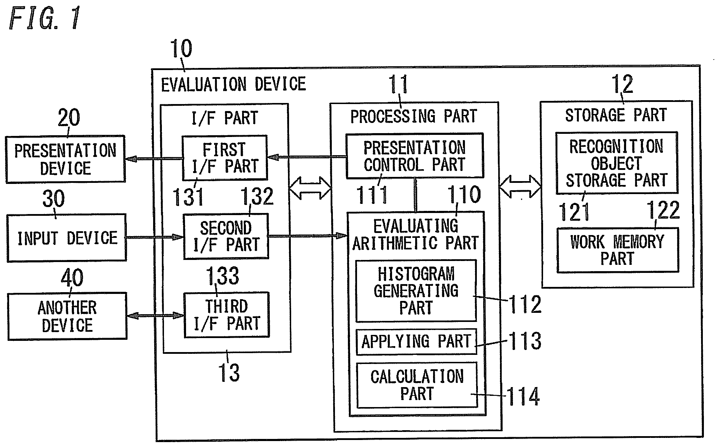

The concentration ratio measurement apparatus described below can objectively measure the concentration ratio on the mental work of the test subject by making the test subject perform the a series of work described below: presenting the recognition object that the test subject is made to recognize, giving the work problem performed about this recognition object, and allowing to input the answer of this work problem. Therefore, as shown in FIG. 1, the concentration ratio measurement apparatus includes a presentation device 20 configured to present recognition objects to the test subject; an input device 30 configured to allow the test subject input the answer of a work problem; and an evaluation device 10 configured to calculate the concentration ratio on the mental work of the test subject. The evaluation device 10 may be a dedicated device. Also, the evaluation device 10 may be realized by executing a program with a general-purpose computer. The computer may be a desktop type or a notebook type. Also, the evaluation device 10 may be a tablet terminal, a smart phone, or a game machine exchangeable a program.

When the computer of the notebook type, the tablet terminal, the smart phone, the game machine, or the like is used as the evaluation device 10, the evaluation device 10 may be integrally provided with the input device 30 at least. Further, the evaluation device 10 may be integrally provided with the presentation device 20 in addition to the input device 30. In short, any of the following three configuration is adopted: a configuration in which the evaluation device 10 is separately provided with the presentation device 20 and the input device 30; a configuration in which the evaluation device 10 is integrally provided with the input device 30; and a configuration in which the evaluation device 10 is integrally provided with the presentation device 20 and the input device 30. When separating the presentation device 20 and the input device 30 from the evaluation device 10, it is also possible to use electronic paper or paper for at least one of the presentation device 20 and the input device 30.

When the input device 30 is separated from the evaluation device 10, a notebook computer, a tablet terminal, a smart phone, a game machine, or the like is used as the input device 30. When a plurality of options are presented by the presentation devicne20, a time when the answer is inputted into the input device 30 is a time when any of the plurality of options is chosen. On the other hand, when the test subject inputs the answer to the input device 30 with a stylus pen (touch pen) that is one of pointing devices, for example, the input device 30 displays, on an area different from an area for inputting the answer, an icon for informing the input device 30 and the evaluation device 10 of the completion of the input. Then, a time when the icon is clicked after the answer is inputted is a time when the answer is inputted to the input device 30.

The recognition object presented on the presentation device 20 is set so that two or more kinds of cognitive elements can be extracted. That is, each of the two or more kinds of cognitive elements means an attribute that the test subject without special knowledge can recognize about a recognition object. It is required that the recognition object includes two or more kinds of attributes described above.

Examples of such the recognition object may include a number, a word, and the like. For example, in the case of the number, the number groups are set, which are obtained by classifying the numbers of 0 to 9 into about three or four groups, and the number with two or more digits (in particular, three or more digits) is used for the recognition object. If classifying to the number groups to which the figure of each digit belongs is performed, the figure of each digit can be used as the cognitive element.

If the recognition object is made into a word, classification of the meaning of a word can be used as the cognitive element, and a character type (form of the character) expressing a word, the number of characters in the word, a sound on a specified position of the word, and a color, a size, and a style of the character, for example, can be also used as the cognitive element. On the other hand, a figure, a sign, and a picture, for example, can be used as the recognition object. About these recognition objects, a form, content, a color, and a size, for example, can be used as the cognitive element.

The work problem demanded in the present embodiment includes: a first work of extracting the cognitive element included in the recognition object; and a second work of choosing the option suitable the cognitive element extracted from the recognition object. As described above, since the recognition object includes two or more kinds of cognitive elements (m kind, m>=2), the first work is a work of extracting two or more kinds of prescribed cognitive elements (n kinds, 2<=n<=m) demanded as the answer among the two or more kinds of cognitive elements. Each of the two or more kinds of prescribed cognitive elements has two or more selections. The second work is a work of choosing the option suitable for the cognitive element extracted in the first work from the options. The number of options is the number of combinations of the two or more selections in each of the two or more kinds of the prescribed cognitive elements.

As for the answer inputted by the test subject inputs into the input device 30, the two or more kinds of selections are set for each cognitive element, and the options of the answer is set with the combination of selections. It is important that the measurement accuracy of the concentration ratio is also suitable. Then, it is required that the cognitive load of the work problem is suitable. In order to give the test subject suitable the suitable cognitive load, about three or four kinds of the cognitive elements are desirable because of the following reason. The cognitive load is too low in two kinds of the cognitive elements. On the other hand, the cognitive load is too high in too many cognitive elements. Therefore, hindrance factor, such as volition deterioration to the work problem, occurs easily.

For example, if three selections are sets for each cognitive element, and the number of cognitive elements is two, nine options (=3×3) is obtained in the combination of two kinds of the cognitive elements (n=2), twenty seven options (=3×3×3) is obtained in the combination of three kinds of the cognitive elements. Therefore, the recognition object is desirably presented so that three or more kinds of cognitive elements are capable of being extracted. If the recognition object is the number or the word described above, the selection of the recognition object is easy, and the kind of the recognition object is also abundant. Therefore, it is possible to measure the concentration ratio so that the bias as to the recognition object does not occur by choosing the recognition object from a large range.

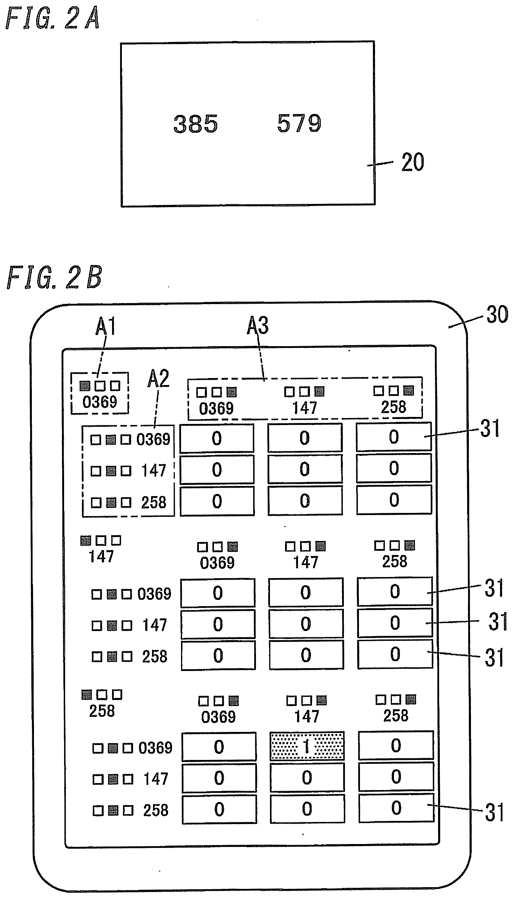

About the case where a recognition object is made into a number, an example of the recognition object shown to the test subject is shown in FIG. 2A, and the display example of the choice into which the test subject inputs the answer is shown in FIG. 2B. As mentioned above, a cognitive element is made into classifying to a number group, and is made into three kinds of number groups of (0369), (147), and (258). Here, only the work divided into the number group to which the figure of each digit of three-digit number belongs is monotonous, and volition may deteriorate by the time when the measurement of the concentration ratio is completed. Therefore, the following is performed: the two sets of three-digit numbers are presented: and combined is the easy four-arithmetical-operations calculation with both numbers so that the volition over work is maintained.

Specifically, the following work is performed as for the pair of the pair of three-digit numbers: the addition of figures of left ends, the multiplication of figures of center, and the subtraction of figures of right ends; and generating three-digit number obtained by arranging figures of one place in order, in a state where the codes of positive/negative of the figures are disregarded. In the example shown in FIG. 2A, the numbers are (385) and (579). Therefore, the addition of the figures of the left ends is 3+5=8. The multiplication of the central is 8×7=56, and the then, "6" is adopted. The subtraction of the right ends is 5-9=-4, and then, "4" is adopted. That is, the number that performs classifying is set to (864).

On the other hand, the figures of three digits are the cognitive element, and the number of cognitive elements is three. Therefore, the combinations of the cognitive elements cannot be indicated with two dimension matrix. For this reason, as shown in FIG. 2B, a group is formed, in which the figures of the left ends are the cognitive elements, and two dimension matrix is formed, in which the figures of the center and the figures of the right ends are the cognitive elements. That is, an example is shown in a region A1, the groups of three kinds of number sets, to which the figures of the left ends belongs, are arranged up and down. Further, as for each group, nine options 31 are set, which are showed as the matrix composed of the combinations of the three kinds of number sets to which the numbers of the center shown in a region A2 as an example, and three kinds of number sets to which the figures of the right ends shown in a region A3 as an example. Therefore, three groups is set, each which includes the nine options 31, and accordingly, twenty nine options in total is presented on one screen.

In the illustrated example, in order to show, as the cognitive element, the figure at which position of the recognition object, three squares are arranged right and left in each of regions A1, A2, and A3, and the positions of the cognitive elements are shown by the squares becoming black. For example, in the region A1, the square of the left end is black, and the figure of the left end is shown to be the cognitive element.

In the example mentioned above, since the work that classifies the triple digits (864) is done, the group of the lower stage by which the left end number is included in (258) is chosen. The position by which a central number is included in (0369) and a right end number is included in (147) within this group is chosen, and the position of a half tone process part currently described as "1" into a choice as a result becomes a correct answer.

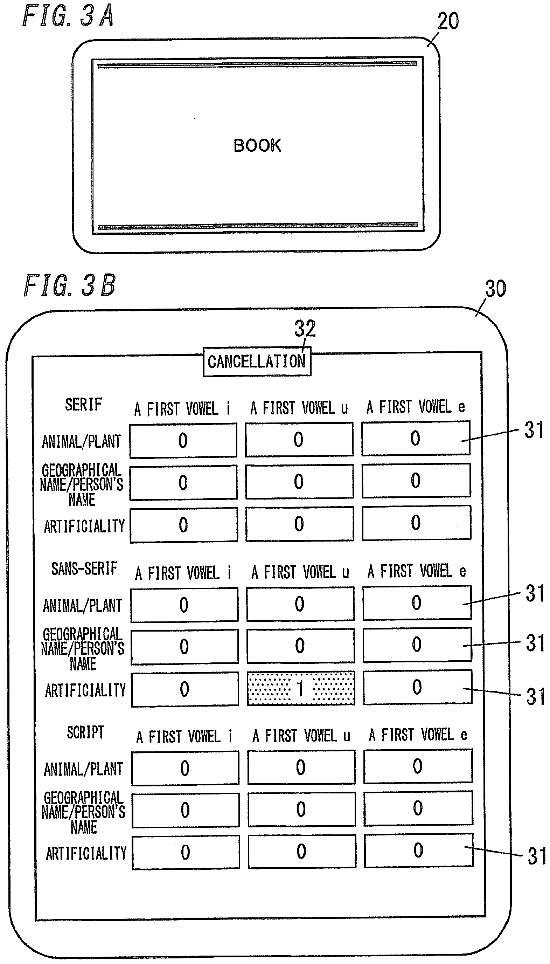

About the case where the recognition object is a word, FIG. 3A shows an example of the recognition object presented to the test subject, and FIG. 3B shows a display example of the options used when the test subject inputs the answer. The cognitive elements are the following three kinds of elements: a font; a first vowel; and a meaning. The selections of the font are serif, sans-serif, and script. The selections of the first vowels are "i", "u", and "e". The selections of the meaning are an animal/plant, a name of a place/person, and artificiality.

In an illustrated example, a recognition object is "BOOK", and the presentation device 20 presents the word "BOOK" with the font of the sans-serif. The cognition element that is a correct answer of this example is (sans-serif, "u", artificiality).

In the example of this embodiment, since the number of cognitive elements is three, as shown in FIG. 3B, a group about one kind of cognitive element is formed, and a two-dimensional matrix is formed for each group about two kinds of remaining cognitive elements. That is, the "character type" of the cognitive elements is divided into the group for each of the selections (Serif, Sans-serif, Script), and then, the nine options 31 are set, which are showed as the two dimension matrix composed of the combination with the "meaning" and the "first vowel" for each group. Therefore, the twenty seven options in total are set on one screen by providing the three groups each which has nine options 31. In the illustrated example, the correct answer is an option 31 of "1" among options 31.

When the recognition object presented to the test subject is Japanese word, a character type may be used instead of the font as the cognitive element. The selections of the character type may be hiragana, katakana, and Chinese character. The number of characters may be used instead of the character type as the cognitive element. The number of characters may be three characters, four characters, and five characters.

Each of the options 31 used for allowing the test subject input the answer is denoted by a rectangular grid. When the test subject chooses any of the options 31, the display made to reverse black and white or the display suitably colored the color is digested. Here, an operation of choosing the option 31 is performed by a movement and a click of a cursor with a pointing device such as a mouse or a trackpad (the depression of a mouse button, tapping of the trackpad, for example). When the touch panel for the input device 30 is used, the operation of choosing the option 31 may be performed by the contact of the finger or a nib to a region as which the option 31 is displayed.

The evaluation device 10 is used with the presentation device 20 and the input device 30, and configured to quantitatively evaluate the concentration ratio about the mental work of the test subject by making the test subject perform the work problem as described above. At least one of the right or wrong of the answer the answering time to the recognition object is used for the evaluation of the concentration ratio.

The evaluation device 10 includes a device including a processor that operates according to a program, and a device for an interface for connecting an external device, as main hardware elements. The device including the processor is selected from a microprocessor, a microcomputer, a DSP (Digital Signal Processor), an FPGA (Field-Programmable Gate Array), or the like. The device for the interface has a function of connecting the presentation device 20 and the input device 30 at least. Further, the device for the interface desirably has a function of communicating through a LAN (Local Area Network) or a WNA (Wide Area Network).

The program executed by the processor may be acquired not only through an electric telecommunication line like Internet, but also by reading a program stored in a readable medium by the computer.

As shown in FIG. 1, the evaluation device 10 includes a processing part 11, a storage part 12, and an interface part 13, in the case of being divided into a functional order. The interface part (hereinafter, referred to as an "I/F part") 13 includes a first I/F part 131 and a second I/F part 132. The presentation device 20 is connected to the first I/F part 131. The input device 30 is connected to the second I/F part 132. The I/F part 13 of the present embodiment includes a third I/F part 133. The third I/F part 133 is connected to the wide area network or local area network represented by the Internet.

The storage part 12 includes a recognition object storage part 121. The recognition object storage part 121 is configured to store the plurality of recognition objects presented to the presentation device 20. The recognition object storage part 121 is configured to store the correct answers that are respectively associated with the recognition objects in addition to the recognition objects. The correct answer is stored in the recognition object storage part 121 in the form of either of the combination of the cognitive element to the recognition object, or the position of the option 31 chosen with the input device 30.

If the case of the example shown in FIGs. 2A and 2B, since the recognition object is (864), the recognition object storage part 121 stores the correct answer in the form of the latter. That is, the stored correct answer is (7, 2). (7, 2) means that the option 31 of the correct answer is a position of the seventh line, and the second row.

In the case of the example shown in FIGs. 3A and 3B, since the recognition object is "BOOK", the recognition object storage part 121 stores (sans-serif, "u", artificiality) as the correct answer in the former form, and stores (5, 2) as the correct answer in the form of the latter.

Although many recognition objects are stored in the recognition object storage part 121, when the concentration ratio is measured, only some recognition objects are presented. The processing part 11 includes a presentation control part 111 configured to choose the plurality of recognition objects used for one measurement among the recognition objects stored in the recognition object storage part 121 and generate one set of the recognition objects including the plurality of recognition objects. The presentation control part 111 has: a function of choosing the recognition objects from the generated set in order, and making the presentation device 20 present the recognition objects; a function of making the presentation device 20 present the options 31 obtained by combining the selections as shown in FIG. 2B and FIG. 3B.

Even if the number of recognition objects stored in the recognition object storage part 121 presents the set of a recognition object in order to the same test subject, and the measurement of the concentration ratio is repeated, the number of recognition objects is set so that the test subject does not get used to the recognition objects. For example, what is necessary is just to be able to generate five to ten sets, using the recognition object stored in the recognition object storage part 121, when 50 to 500 recognition objects shall be the one set. Any recognition objects may overlap and be used between the sets.

If the composition that communicates, through the third I/F part 133, with another device 40, which is different from the evaluation device 10 and the presentation device 20 such as a server, is adopted, the set of the recognition objects can be transmitted from the device 40. Therefore, it is possible to reduce a storage capacity required for the recognition object storage part 121, and update the recognition object suitably. Also, it is possible to provide, to the device 40, the main functions of the evaluation device 10, and provide, to a side of the test subject, only the presentation device 20, the input device 30, and the I/F part 13.

The recognition object of the number equivalent to two or more sets is stored in the recognition object storage part 121, and the presentation control part 111 extracts suitably the recognition object stored in the recognition object storage part 121, and generates the one set of the recognition objects. When the test subject inputs the answer into the input device 30, the presentation control part 111 makes the presentation device 20 present the following recognition object instead of the recognition object under presentation.

The storage part 12 includes a work memory part 122. The work memory part 122 is configured to store, for each recognition object, the time period from a time when the recognition object is presented on the presentation device 20 to a time when the answer is inputted into the input device 30. The work memory part 122 is also configured to store the right or wrong of the answer in addition to the answering time for each recognition object. That is, when the test subject inputs the answer into the input device 30 in a state where the recognition object is presented on the presentation device 20, the work memory part 122 is configured to store the answering time and the right or wrong of the answer by the test subject.

After the answers about the one set of the recognition objects are obtained, the processing part 11 is configured to calculate an answer rate of the set. The work memory part 122 is configured to store the answer rate. In the above-mentioned example, the work memory part 122 is configured to store both of the answering time and the right or wrong of the answer for each recognition object. However, the work memory part 122 may be configured to store only the answering time. Hereinafter, the answering time for each recognition object and the right or wrong of the answer for each recognition object are referred to as work information. That is, the work memory part 122 is configured to store, as the work information, at least one of the answering time and the right or wrong of the answer for each recognition object.

Here, as shown in FIG. 3B, the "cancellation" button 32 that cancels information just before stored on the work memory part 122 may be added on the screen of the input device 30 in addition to the grid used as the options 31. If the "cancellation" button 32 is operated, the presentation control part 111 is configured to eliminate the information just before stored in the work memory part 122, and make the presentation device 20 present the last recognition object.

In this state, when the option 31 corresponding to the recognition object presented on the presentation device 20 is chosen by the test subject, the information about this recognition object is stored in the work memory part 122. The answering time stored in the work memory part 122 is a time period of the sum of from a time when the same recognition object is presented on the presentation device 20 first to a time when the answer is inputted into the input device 30 after the operation to the "cancellation" button 32. The right or wrong of the answer stored in the work memory part 122 becomes right or wrong to the answer chosen after the operation to the "cancellation" button 32.

Although the "cancellation" button 32 is shown in FIG. 3B, when the test subject cannot choose the choice to a recognition object, it may be notified, to the presentation control part 111, to shift to the following recognition object by providing a skip button. When the test subject operates the "cancellation" button 32 and a skip button, using for evaluation of the difficulty of work problem is possible by storing a history.

Incidentally, the presentation control part 111 may have the function to summarize the plurality of recognition objects to the presentation device 20, and to make them show. When making the presentation device 20 present the plurality of recognition objects collectively, time until a choice is chosen first to two or more displayed recognition objects is made into one answering time, and time until the following choice is chosen should just be henceforth made into answering time. That is, what is necessary is to measure elapsed time after the presentation device 20 presents the plurality of recognition objects, and just to apply the time divided whenever a choice is chosen to the answering time over each recognition object.

As shown in FIG. 1, the processing part 11 includes an evaluating arithmetic part 110. The evaluating arithmetic part 110 is configured to calculate the evaluation value about the concentration ratio of the test subject. After the presentation device 20 presents the one set of the recognition objects, and the work memory part 122 stores information that the test subject inputs into the input device 30, the evaluating arithmetic part 110 calculates the evaluation value about the concentration ratio of the test subject using the statistics value of the work information stored in the work memory part 122.

Hereinafter, the processing that the evaluating arithmetic part 110 performs will be described. The work adopted by the present embodiment cannot be easily affected by influence of an experience effect or knowledge, and moreover, since dispersion in difficulty is small, it is possible to objectively extract the evaluation value about the concentration ratio from work information. The work information is at least one of the answering time and the right or wrong of the answer. It is easy to measure the work information quantitatively. Also, since the work information is measured using comparatively many options in the work, the resolution about the concentration ratio is high.

The evaluating arithmetic part 110 is configured to convert the work information stored in the work memory part 122 into a form in which the evaluation value on the concentration ratio of the test subject can be calculated. Since it is considered that each of two or more pieces of work information has dispersion for recognition objects, the evaluation value on the concentration ratio is calculated by using the statistics value of the work information. When the answering time is used as the work information, a frequency distribution, for example, may be used. When the right or wrong of the answer is used as the work information, an answer rate in the unit time, for example, may be used. When both of the answering time and the right or wrong of the answer as the work information are used the work information, a frequency distribution is calculated by multiplying the frequency for each unit time by the answer rate calculated for each unit time, and just to calculate.

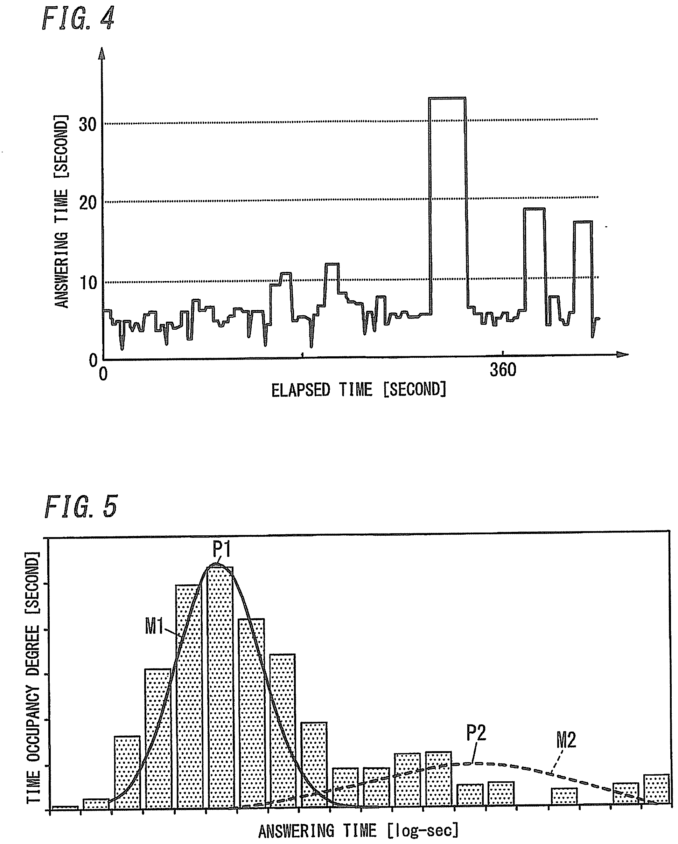

Hereinafter, the case where the evaluation value about the concentration ratio calculated using the answering time will be described. That is, the evaluating arithmetic part 110 calculates the frequency distribution about a set of the answering time acquired to the one set of the recognition objects, and uses for the evaluation value about the concentration ratio the characteristic quantity extracted from the frequency distribution. FIG. 4 shows an example in which the answering time stored in the work memory part 122 is expressed so as to be associated with the elapsed time from the start of one measurement. A horizontal axis in FIG. 4 expresses the elapsed time from the measurement start, and a vertical axis expresses the answering time for each recognition object. In the illustrated example, when a period (left end part) in which the elapsed time from starting the measurement is short compare with a period (right end part) in which the elapsed time is long, the answering time of the period (left end part) in which the elapsed time is short tend to be longer than the period (right end part) in which the elapsed time is long. This tendency is considered to mean that activation is declining by monotonous feeling, fatigue, deterioration of vigilance, deterioration of motivation, for example, of the test subject.

Incidentally, in the state where a person performs the intellectual work, the model described using three states of a "working state", a "short-term rest state" and a "long-term rest state" is considered. The "working state" is a state where the cognitive resources are assigned to the target (task target) and the processing of the work advances. The "short-term rest state" is a state where the processing of the work is stopped during the short-time unconsciously although the cognitive resources are assigned to the target. This state is physiologically generated in fixed probability. The "long-term rest state" is a state where the cognitive resources are not assigned to the target and the rest is taken during the long time.

The "working state" and the "short-term rest state" can be considered to be the concentration state since the cognitive resources are assigned to the target. The "long-term rest state" can be considered to be the non-concentration state since the cognitive resource is not assigned to the target. In order to evaluate the concentration state and the non-concentration state quantitatively, the frequency distribution is calculated paying attention to the answering time stored in the work memory part 122. As a result, the time occupancy degree histogram as shown in FIG. 5 is obtained.

The time occupancy degree histogram is a histogram that denotes, as a time occupancy degree, a ratio of a total of a answering time for each section to the total of the answering time of all sections (measurement period) when the answering time are divided into two or more sections. A horizontal axis expresses the answering time with logarithmic scale, and a vertical axis expresses the time occupancy degree. The result that the time occupancy degree histogram at the time of performing the above work becomes bimodal with two peaks is obtained as shown in FIG. 5.

In this time occupancy degree histogram, it is considered that a first mountain-shaped part M1 with a peak where the answering time is shorter is a state in which the "working state" and the "short-term rest state" are mixed. It is considered that a second mountain-shaped part M2 with a peak where the answering time is longer are a state in which the "working state", the "short-term rest state", and the "long-term rest state" are mixed. The model mentioned above in order to interpret the time occupancy degree histogram obtained based on answering time when the work mentioned above is done is used for the present embodiment.

Since each of the first peak P1 (first mountain-shaped part M1) and the second peak P2 (second mountain-shaped part M2) has the form resembled the log normal distribution, it is guessed that the time occupancy degree histogram described above is capable of being applied to a superimposition of two probability density functions of log normal distributions. In the example shown in FIG. 5, since the time occupancy degree of the first peak P1 is larger than that of the second peak P2, the first peak P1 is a peak at which the time occupancy degree is the largest, and the second peak P2 is a peak at which the time occupancy degree is the next large. That is, the first mountain-shaped part M1 has the first peak P1, and the second mountain-shaped part M2 has the second peak P2.

Here, the probability density function of the log normal distribution applied to the first mountain-shaped part M1 is set to f1(t). The probability density function of the log normal distribution applied to the second mountain-shaped part M2 is set to f2(t). The function f(t) showing the time occupancy degree histogram is denoted by f(t)=f1(t)+f2(t). Here, the expected value of the function f1(t) is set to E, and the number of answers included in the first mountain-shaped part M1 is temporarily set to N1, and the number of answers included in the second mountain-shaped part M2 is temporarily set N2.

An area S1 equivalent to the first mountain-shaped part M1 is denoted by S1=E×N1. As described above, it is assumed that the second mountain-shaped part M2 expresses the state where the "working state", the "short-term rest state", and the "long-term rest state" are mixed. Therefore, it is considered that an area S2 equivalent to the "short-term rest state" and the "working state" of the area of the second mountain-shaped part M2 is denoted by S2=E×N2.

Since N1+N2 is a total of the number of answers, if N1+N2 is set to N1+N2=N, in the time occupancy degree histogram, the gross area S equivalent to the "working state" and the "short-term rest state" is expressed as S=S1+S2=E×N. The calculated total area S is equivalent to the total time (concentration time) of the concentration state. That is, the ratio of the total area S of the answering time to the total (measurement period) can be used as the evaluation value that evaluates the concentration ratio quantitatively. If the total area S is subtracted from the measurement period, the total time (non-concentration time) of the non-concentration state is found.

In order to calculate the evaluation value of the concentration ratio as described above, it is necessary to determine that the parameters of the functions f1(t) and f2(t) apply to the time occupancy degree histogram. Since both of the functions f1(t) and f2(t) are the probability density functions of the log normal distributions, parameters (average values and distributions) about the functions f1(t) and f2(t) are optimized so as to be suitable for the function f(t).

Since the solution space for searching for the parameters optimal about the functions f1(t) and f2(t) is vast, it is necessary that the maximum likelihood values of the parameters are calculated using the well-known algorithm like the EM algorithm. The parameters of the functions f1(t) and f2(t) are converged in comparatively short time, if the initial values are set appropriately, but the change of the initial values are repeated until the initial values are changed and the parameters complete, in not converging.

In order to calculate the evaluation value of the concentration ratio as described above, as shown in FIG. 1, the evaluating arithmetic part 110 includes: a histogram generating part 112 configured to generate the time occupancy degree histogram; and an applying part 113 configured to apply the function to the time occupancy degree histogram. The evaluating arithmetic part 110 further includes a calculation part 114 configured to calculate the concentration time or the concentration ratio using the parameter of the applied function.

As described above, the applying part 113 is configured to apply the two probability density functions f1(t) and f2(t) of the log normal distributions to the time occupancy degree histogram. That is, the applying part 113 is configured to approximate the time occupancy degree histogram as a superimposition of the probability density function f1(t) of the first log normal distribution applied to the first mountain-shaped part M1(t), and the probability density function f2(t) of the second log normal distribution applied to the second mountain-shaped part M2(t). The calculation part 114 is configured to extract, as the feature amount, the expected value calculated from the probability density function f1(t) of the first log normal distribution. Then, the calculation part 114 is configured to calculate, as the concentration time, the product of this feature amount and the total of the answers. The calculation part 114 is configured to calculate, as the evaluation value equivalent to the concentration ratio, the ratio of the calculated concentration time to the measurement period. The calculation part 114 may be configured to calculate, as the non-concentration time, the value obtained by subtracting the calculated concentration time from the measurement period, and may be configured to calculate, as the evaluation value equivalent to the non-concentration ratio, a ratio of the non-concentration time to the measurement period.

The embodiment described above described while the case is assumed where the presentation device 20 presents the questions by one in order. However, as described above, the answering time can also be measured by using the times between the answers. For example, the time between the time when inputting of the answer of one question (first question) into the input device 30 is finished, and the time when inputting of the answer of the following one question (second question) is finished may be used for the answering time of one question (first question). That is, the time period from the time when the input of the answer of the first question is finished to the time when the input of the answer of the following one question (second question) is finished may be used as the answering time of the second question. When making the answering time into the time during the answer, it is possible to find the answering time, without using the presentation device 20. That is, the answering time is found if the time when a question expressed in paper or the like is presented to the test subject, and makes the answer input into the input device 30 and when the answer finished being input for each question is stored. When the questions described in paper or the like are presented to the test subject, the question does not need to be presented to one sheet by list and may be presented in each one sheet by one question.

### (Second embodiment)

Although the case where a three-state model is used was described in the first embodiment, an example using simpler two state models will be described in the present embodiment.

Here, a state where the answering time is in a specified base period is referred to as the "break state". A state where the answering time exceeds the base period is referred to as the "working state". The working state may be put in another way as the concentration state, and the break state may be put in another way as the non-concentration state. Transition with the working state and the break state is denoted, for example, by a Markov model. It is considered that the break state is further divided into two steps according to the time length of the break state. However, it is assumed the Markov model of two states of the working state and the break state. If such a model is assumed, it is possible to obtain the assumption that the frequency distribution of the answering time reflects two states of the working state and the break state.

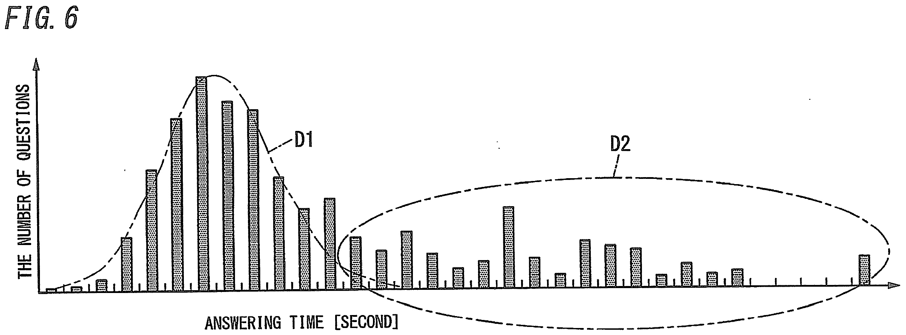

In order to verify this assumption, the frequency distribution is calculated paying attention to the answering time stored in the work memory part 122, and the histogram in which the time-axis is logarithm is produced. Producing of the histogram obtains the following result: the region D1 is substantially applied to the log normal distribution; and the region D2 is not applied to the log normal distribution, as shown in FIG. 6. That is, the result is obtained, in which, although the region D1 where the answering time is comparatively short is applied to one log normal distribution, the region D2 where the answering time is comparatively long is not applied to this log normal distribution. It is considered that the region D1 that is substantially applied to the log normal distribution is the working state where the concentration ratio is comparatively high. Since the region D2 that is not applied to the log normal distribution is comparatively the long answering time, it is considered that the region D2 is equivalent to the break state. In this model, like the model of the first embodiment, there are two bell type regions, and it is considered that each of the regions D1 and D2 is applied to the log normal distribution.

Then, a base period is set to answering time, and if answering time considers among histograms that the region that is in the base period defined suitably is the region of the working state, it is possible to use for the evaluation value about the concentration ratio by making the parameter of this region into characteristic quantity. A base period is set near the maximum of the region D1 applicable to the log normal distribution. The region D2 that is not applied to the log normal distribution is equivalent to the region of the break state.

In the first embodiment, the present embodiment is different from the first embodiment in the following point. In the first embodiment, it is considered that the state of the test subject in the region D2 is a state where the "working state", the "short-term rest state", and the "long-term rest state" are mixed. In the present embodiment, it is considered that the state of the test subject in the region D2 is the "long-term rest state". That is, it considered that the state of the test subject in the region D1 is the working state (concentration state), and it is considered that the state of the test subject in the region D2 is the break state (non-concentrating state).

Here, it is assumed that the histogram about the answering time when the time-axis is a logarithm is applied to the log normal distribution. Therefore, the average value µ and the standard deviation σ are calculated from the region equivalent to the working state. Peak value α of frequency equivalent to the working state is calculated. By using these parameters (µ, σ, a), characteristic quantity is obtained from the region of the working state as follows.

That is, the standard deviation σ or the ratio (=α/σ) of the peak value α to the standard deviation σ in the region D1 equivalent to the working state expresses a kurtosis of the region D2 equivalent to the working state. The kurtosis of the form of this region D2 reflects the concentration ratio. Therefore, the standard deviation σ or the ratio α/σ is calculated as the evaluation value about the concentration ratio.

Incidentally, the method of asking for a parameter (µ, σ, α) from the histogram of answering time as shown in FIG. 6 cannot be formulized. Therefore, various combination of a parameter is generated and the parameter that is best applied to a histogram is chosen. Since huge time is required when extracting a parameter if a round robin algorithm is used in order to generate various combination of a parameter, it is desirable to use for selection of a parameter EM algorithm mentioned above or a genetic algorithm.

As described above, the evaluating arithmetic part 110 is configured to calculate the frequency distribution of the answering time using the answering time stored in the work memory part 122, and extract the region equivalent to the working state about the histogram made into the logarithm axis the time-axis of this frequency distribution. The evaluating arithmetic part 110 is configured to calculate the evaluation value about the concentration ratio by using the parameter of the region equivalent to the working state. The histogram generated by the evaluating arithmetic part 110, the parameters (µ, σ, α) obtained from the histogram, and the evaluation value calculated from the histogram are displayed on the display device that serves as the presentation device 20 if needed. The other configurations and operations are the same as those of the first embodiment.

When the evaluation device 10 communicates with another device 40, such a server, via the third I/F part 133, it is possible by transmitting a question to the recognition object storage part 121 from the device 40 to update a question as required. The function of the evaluation device 10 may be provided to the device 40, such as a server, and the device 40 and a device including the presentation device 20, the input device 30, and the I/F part 13 may constitute the intellectual-productivity analysis apparatus.

The method of computing the evaluation value about the concentration ratio from the work information that the work memory part 122 has stored in the evaluating arithmetic part 110 is not limited to the above-mentioned method. For example, in the example described above, although the working state is paid attention in the histogram, it is possible to adopt the technology separated into the working state and the break state. The right or wrong of the answer may be used as the work information instead of the answering time. Alternatively, both of the answering time and the right or wrong of the answer may be used as the work information.

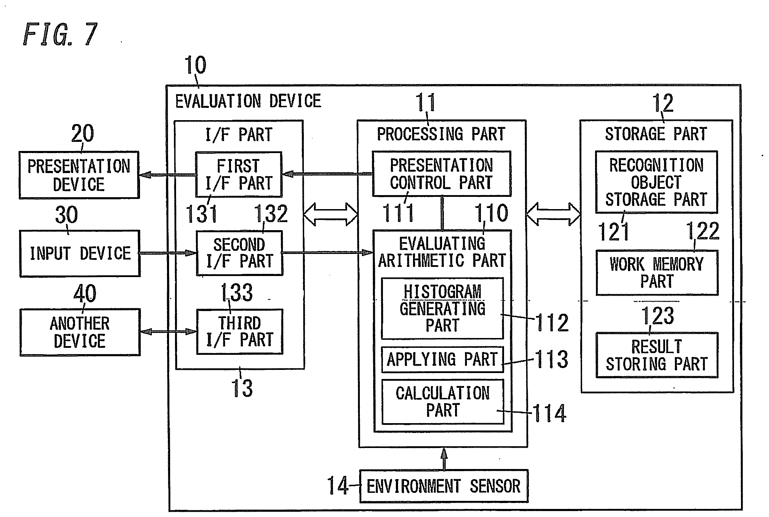

Incidentally, it is predicted that the concentration ratio of the test subject is affected by the influence of the environmental elements, such as illumination of working clearance, temperature, humidity, noise, and a bad smell. In order to verify this prediction, it is necessary to measure the relation between the environmental element and the concentration ratio. Then, as shown in FIG. 7, the evaluation device 10 is provided with the environment sensor 14 that measures at least one kind in the environmental element in which influencing the concentration ratio is predicted. It is not indispensable that the environment sensor 14 is attached to the evaluation device 10, and the composition with which the environment sensor 14 is connected to the I/F part 13 may be adopted.

When the evaluating arithmetic part 110 is computing the evaluation value about the concentration ratio here using the statistics value of answering time and change arises in the environmental element while measuring the concentration ratio, it is impossible to distinguish whether the change of the evaluation value depends on the change of the concentration ratio or the change of the environmental element. Therefore, while having shown the one set of the recognition objects, it is necessary to fix the environmental element. In order to store in what kind of environment the work information is acquired, the environmental element measured by the environment sensor 14 is stored in the result storing part 123 while being associated with the evaluation value calculated by the evaluating arithmetic part 110.

The result storing part 123 is configured to store the evaluation value about the concentration ratio with the environmental element for each test subject. Therefore, if the information stored in the result storing part 123 is used, it is possible to evaluate how the concentration ratio is affected by the influence of the environmental element.

It is assumed also when the environment sensor 14 detects that the environmental element deviated from prescribed tolerance level, and changed in the period that has presented the one set of the recognition objects. Then, the composition that matches with the answering time the environmental element that the environment sensor 14 measured, and is stored in the work memory part 122 in the period that has presented the one set of the recognition objects if the environmental element that the environment sensor 14 measured deviates from tolerance level may be adopted. If this composition is adopted, it is possible to distinguish and store the answering time for each environmental element in the period that has presented the one set of the recognition objects.

It enables the evaluating arithmetic part 110 to detect change of the concentration ratio according to the environmental element by calculating the evaluation value about the concentration ratio for each environmental element. However, as for change of the environmental element, when changing the environmental element while having shown the one set of the recognition objects since deterioration of the concentration ratio by fatigue arises if the number of recognition objects included in the one set increases, it is desirable to stop to about two or three times.

According to the embodiment mentioned above, the evaluation value about the concentration ratio is calculated from the work information, and the individual difference of the concentration ratio is not taken into consideration. What is necessary is just to relativize change of the concentration ratio for each environmental element for each individual by storing the history of the answering time for each test subject, and computing the evaluation value about the concentration ratio calculated from the answering time for each environmental element, when taking into consideration the individual difference of the concentration ratio. Thus, not using the absolute value of the evaluation value about the concentration ratio, it is possible by using the relative value over a specific environmental element to reduce the individual difference of the influence of the environmental element.

## Claims

1. A concentration ratio measurement apparatus comprising:
a presentation device (20) configured to present a plurality of recognition objects to a test subject;
an input device (30) configured to allow the test subject to input an answer of a work problem that includes a mental work of object recognition, for each of the plurality of recognition objects; and
an evaluation device (10) configured to evaluate a concentration ratio of the test subject on the mental work using at least one of an answer rate and an answering time from a time when the recognition object is presented on the presentation device (20) to a time when the answer is inputted into the input device (30), for each of the plurality of recognition objects;
wherein the work problem comprises:
a work of extracting two or more kinds of prescribed cognitive elements included in the recognition object, for each of the plurality of recognition objects, and
a work of choosing, from a plurality of options (31), an option suiting to the two or more prescribed cognitive elements extracted from the recognition object, for each of the plurality of recognition objects, a number of the plurality of options (31) being a number of combinations of selections set to each of the two or more kinds of prescribed cognitive elements; and
wherein the evaluation device (10) comprises:
a recognition object storage part (121) configured to store the plurality of recognition objects,
a presentation control part (111) configured to perform a function of making the presentation device (20) present the plurality of recognition objects stored in the recognition object storage part (121), and a function of making the presentation device (20) present the plurality of options (31) for each of the plurality of recognition objects,
a work memory part (122) configured to store, as work information, the at least one of the answering time and the answer rate for each of the plurality of recognition objects, and
an evaluating arithmetic part (110) configured to calculate an evaluation value of the concentration ratio using a statistics value of the work information stored in the work memory part (122) while the plurality of recognition objects are presented on the presentation device (20);
wherein the evaluating arithmetic part (110) comprises:
a histogram generating part (112) configured to classify the answering time into a plurality of sections, the histogram generating part (112) being configured to regard, as a time occupancy degree, a ratio of a total of the answering time in a section to a total of the answering time for each of the plurality of sections, the histogram generating part (112) being configured to generate a time occupancy degree histogram expressing a distribution of the time occupancy degree,
an applying part (113) configured to approximate the time occupancy degree histogram as a superimposition of a probability density function of a first log normal distribution, which is applied to a first mountain-shaped part with a first peak part in which the time occupancy degree is the largest, and a probability density function of a second log normal distribution, which is applied to a second mountain-shaped part with a second peak part in which the time occupancy degree is the next largest, and
a calculation part (114) configured to extract, as a feature amount, an expected value calculated from the probability density function of the first log normal distribution, and calculate, as a concentration time, a product of the feature amount and a total number of answers, the calculation part (114) being configured to calculate, as the evaluation value of the concentration ratio, a ratio of the concentration time to a measurement period that is a total of the answering time.

2. The concentration ratio measurement apparatus according to claim 1, wherein the calculation part (114) is configured to calculate, as a non-concentration time in the measurement period, a value obtained by subtracting the concentration time from the measurement period.

3. The concentration ratio measurement apparatus according to claim 1 or 2,
wherein each of the plurality of recognition objects is three or more-digit number,
wherein each of the two or more kinds of prescribed cognitive elements is a figure of each digit of the number,
wherein the selections are number sets including two or more figures, the number sets are divided into three or more kinds of groups, and
wherein the work is a work of classifying each of the two or more kinds of prescribed cognitive elements into a number set to which each of the two or more kinds of prescribed cognitive elements belongs.

4. The concentration ratio measurement apparatus according to claim 1 or 2, wherein each of the plurality of recognition objects is a word presented so that three or more kinds of cognitive elements is extractable as the two or more kinds of prescribed recognition elements.

5. The concentration ratio measurement apparatus according to any one of claims 1 to 4, further comprising:
an environment sensor (14) configured to measure at least one of two or more kinds of environmental elements that influences the concentration ratio in an environment of the test subject; and
a result storing part (123) configured to store an environmental element measured by the environment sensor (14) so as to be associated with the evaluation value calculated by the evaluating arithmetic part (110).

6. The concentration ratio measurement apparatus according to any one of claims 1 to 5, wherein the presentation device (20) and the input device (30) are integrally provided with the evaluation device (10).

7. A program to function a computer as an evaluation device (10) configured to make a presentation device (20) present a plurality of recognition objects and regard, as a work problem, a work of extracting two or more kinds of prescribed cognitive elements included in a recognition object for each of the plurality of recognition objects; and
a work of choosing, from a plurality of options (31), an option suiting to the two or more prescribed cognitive elements extracted from the recognition object for each of the plurality of recognition objects, a number of the plurality of options (31) being a number of combinations of selections set to each of the two or more kinds of prescribed cognitive elements, the evaluation device (10) being configured to allow a test subject input, from an input device (30), an answer of a work problem that includes a mental work of object recognition for each of the plurality of recognition objects, the evaluation device (10) being configured to evaluate a concentration ratio of the test subject on the mental work using at least one of an answer rate and an answering time from a time when the recognition object is presented on the presentation device (20) to a time when the answer is inputted into the input device (30), for each of the plurality of recognition objects;
wherein the computer functioning as the evaluation device (10) comprises:
a recognition object storage part (121) configured to store the plurality of recognition objects,
a presentation control part (111) configured to perform a function of making the presentation device (20) present the plurality of recognition objects stored in the recognition object storage part (121); and a function of making the presentation device (20) present the plurality of options (31) for each of the plurality of recognition objects,
a work memory part (122) configured to store, as work information, the at least one of the answering time and the answer rate for each of the plurality of recognition objects, and
an evaluating arithmetic part (110) configured to calculate an evaluation value of the concentration ratio using a statistics value of the work information stored in the work memory part (122) while the plurality of recognition objects are presented on the presentation device (20);
wherein the evaluating arithmetic part (no) comprises:
a histogram generating part (112) configured to classify the answering time into a plurality of sections, the histogram generating part being configured to regard, as a time occupancy degree, a ratio of a total of the answering time in a section to a total of the answering time for each of the plurality of sections, the histogram generating part being configured to generate a time occupancy degree histogram expressing a distribution of the time occupancy degree;
an applying part (113) configured to regard the time occupancy degree histogram as a superimposition of a probability density function of a first log normal distribution, which is applied to a first mountain-shaped part with a first peak part in which the time occupancy degree is the largest, and a probability density function of a second log normal distribution, which is applied to a second mountain-shaped part with a second peak part in which the time occupancy degree is the next largest; and
a calculation part (114) configured to extract, as a feature amount, an expected value calculated from the probability density function of the first log normal distribution, and calculate, as a concentration time, a product of the feature amount and a total number of the answer, the calculation part (114) being configured to calculate, as the evaluation value, a ratio of the concentration time to a measurement period that is a total of the answering time.

## Patentansprüche

1. Konzentrationsverhältnis-Messvorrichtung, umfassend:
eine Präsentationsvorrichtung (20), die konfiguriert ist, um einer Testperson mehrere Erkennungsobjekte zu präsentieren;
eine Eingabevorrichtung (30), die konfiguriert ist, um es der Testperson zu ermöglichen, für jedes der mehreren Erkennungsobjekte eine Antwort auf ein Arbeitsproblem einzugeben, das eine gedankliche Arbeit der Objekterkennung beinhaltet; und
eine Bewertungsvorrichtung (10), die konfiguriert ist, um ein Konzentrationsverhältnis der Testperson auf der gedanklichen Arbeit unter Verwendung einer Antwortrate und/oder einer Antwortzeit von einem Zeitpunkt, zu dem das Erkennungsobjekt auf der Präsentationsvorrichtung (20) präsentiert wird, bis zu einem Zeitpunkt, zu dem die Antwort in die Eingabevorrichtung (30) eingegeben wird, für jedes der mehreren Erkennungsobjekte zu bewerten;
wobei das Arbeitsproblem umfasst:
eine Arbeit des Extrahierens von zwei oder mehr Arten von vorgeschriebenen kognitiven Elementen, die in dem Erkennungsobjekt beinhaltet sind, für jedes der mehreren Erkennungsobjekte, und
eine Arbeit des Auswählens, aus mehreren Optionen (31), einer Option, die zu den zwei oder mehr vorgeschriebenen kognitiven Elementen passt, die aus dem Erkennungsobjekt extrahiert werden, für jedes der mehreren Erkennungsobjekte, wobei eine Anzahl der mehreren Optionen (31) eine Anzahl von Kombinationen von Auswahlen ist, die für jede der zwei oder mehr Arten von vorgeschriebenen kognitiven Elementen eingestellt sind; und
wobei die Auswertungsvorrichtung (10) umfasst:
einen Erkennungsobjektspeicherteil (121), der konfiguriert ist, um die mehreren Erkennungsobjekte zu speichern,
einen Präsentationssteuerteil (111), der konfiguriert ist, um eine Funktion des Veranlassens der Präsentationsvorrichtung (20), die mehreren Erkennungsobjekte zu präsentieren, die in dem Erkennungsobjektspeicherteil (121) gespeichert sind, und eine Funktion des Veranlassens der Präsentationsvorrichtung (20), die mehreren Optionen (31) für jedes der mehreren Erkennungsobjekte zu präsentieren, durchzuführen,
einen Arbeitsspeicherteil (122), der konfiguriert ist, um als Arbeitsinformationen die Antwortzeit und/oder die Antwortrate für jedes der mehreren Erkennungsobjekte zu speichern, und
einen arithmetischen Bewertungsteil (110), der konfiguriert ist, um einen Bewertungswert des Konzentrationsverhältnisses unter Verwendung eines Statistikwerts der Arbeitsinformationen zu berechnen, die in dem Arbeitsspeicherteil (122) gespeichert sind, während die mehreren Erkennungsobjekte auf der Präsentationsvorrichtung (20) präsentiert werden;
wobei der arithmetische Bewertungsteil (110) umfasst:
einen Histogrammerzeugungsteil (112), der konfiguriert ist, um die Antwortzeit in mehrere Abschnitte zu klassifizieren, wobei der Histogrammerzeugungsteil (112) konfiguriert ist, um als einen Zeitbelegungsgrad ein Verhältnis einer Summe der Antwortzeit in einem Abschnitt zu einer Summe der Antwortzeit für jeden der mehreren Abschnitte zu betrachten, wobei der Histogrammerzeugungsteil (112) konfiguriert ist, um ein Zeitbelegungsgradhistogramm zu erzeugen, das eine Verteilung des Zeitbelegungsgrads ausdrückt,
einen Anwendungsteil (113), der konfiguriert ist, um das Zeitbelegungsgradhistogramm als eine Überlagerung einer Wahrscheinlichkeitsdichtefunktion einer ersten Lognormalverteilung, die auf einen ersten bergförmigen Teil mit einem ersten Spitzenteil angewendet wird, in dem der Zeitbelegungsgrad am größten ist, und einer Wahrscheinlichkeitsdichtefunktion einer zweiten Lognormalverteilung, die auf einen zweiten bergförmigen Teil mit einem zweiten Spitzenteil angewendet wird, in dem der Zeitbelegungsgrad am nächstgrößten ist, anzunähern, und
einen Berechnungsteil (114), der konfiguriert ist, um als eine Merkmalsmenge einen erwarteten Wert zu extrahieren, der aus der Wahrscheinlichkeitsdichtefunktion der ersten Lognormalverteilung berechnet wird, und als eine Konzentrationszeit ein Produkt der Merkmalsmenge und einer Gesamtzahl von Antworten zu berechnen, wobei der Berechnungsteil (114) konfiguriert ist, um als den Bewertungswert des Konzentrationsverhältnisses ein Verhältnis der Konzentrationszeit zu einer Messperiode zu berechnen, die eine Summe der Antwortzeit ist.

2. Konzentrationsverhältnis-Messvorrichtung nach Patentanspruch 1, wobei der Berechnungsteil (114) konfiguriert ist, um als eine Nicht-Konzentrationszeit in der Messperiode einen Wert zu berechnen, der durch Subtrahieren der Konzentrationszeit von der Messperiode erhalten wird.

3. Konzentrationsverhältnis-Messvorrichtung nach Patentanspruch 1 oder 2,
wobei jedes der mehreren Erkennungsobjekte eine drei- oder mehrstellige Zahl ist,
wobei jede der zwei oder mehr Arten von vorgeschriebenen kognitiven Elementen eine Figur jeder Ziffer der Zahl ist,
wobei die Auswahlen Zahlensätze sind, die zwei oder mehr Figuren beinhalten, wobei die Zahlensätze in drei oder mehr Arten von Gruppen unterteilt sind, und
wobei die Arbeit eine Arbeit des Klassifizierens jeder der zwei oder mehr Arten von vorgeschriebenen kognitiven Elementen in einen Zahlensatz ist, zu dem jede der zwei oder mehr Arten von vorgeschriebenen kognitiven Elementen gehört.

4. Konzentrationsverhältnis-Messvorrichtung nach Patentanspruch 1 oder 2, wobei jedes der mehreren Erkennungsobjekte ein Wort ist, das so präsentiert wird, dass drei oder mehr Arten von kognitiven Elementen als die zwei oder mehr Arten von vorgeschriebenen Erkennungselementen extrahierbar sind.

5. Konzentrationsverhältnis-Messvorrichtung nach einem der Patentansprüche 1 bis 4, ferner umfassend:
einen Umgebungssensor (14), der konfiguriert ist, um mindestens eine von zwei oder mehr Arten von Umgebungselementen zu messen, die das Konzentrationsverhältnis in einer Umgebung der Testperson beeinflussen; und einen Ergebnisspeicherteil (123), der konfiguriert ist, um ein Umgebungselement zu speichern, das durch den Umgebungssensor (14) gemessen wird, um mit dem Bewertungswert verknüpft zu werden, der durch den arithmetischen Bewertungsteil (110) berechnet wird.

6. Konzentrationsverhältnis-Messvorrichtung nach einem der Patentansprüche 1 bis 5, wobei die Präsentationsvorrichtung (20) und die Eingabevorrichtung (30) integral mit der Bewertungsvorrichtung (10) vorgesehen sind.

7. Programm, um einen Computer als eine Bewertungsvorrichtung (10) zu betreiben, die konfiguriert ist, um eine Präsentationsvorrichtung (20) zu veranlassen, mehrere Erkennungsobjekte zu präsentieren und als ein Arbeitsproblem eine Arbeit des Extrahierens von zwei oder mehr Arten von vorgeschriebenen kognitiven Elementen, die in einem Erkennungsobjekt beinhaltet sind, für jedes der mehreren Erkennungsobjekte zu betrachten; und
eine Arbeit des Auswählens, aus mehreren Optionen (31), einer Option, die zu den zwei oder mehr vorgeschriebenen kognitiven Elementen passt, die aus dem Erkennungsobjekt extrahiert werden, für jedes der mehreren Erkennungsobjekte, wobei eine Anzahl der mehreren Optionen (31) eine Anzahl von Kombinationen von Auswahlen ist, die für jede der zwei oder mehr Arten von vorgeschriebenen kognitiven Elementen eingestellt sind, wobei die Bewertungsvorrichtung (10) konfiguriert ist, um es einer Testperson zu ermöglichen, von einer Eingabevorrichtung (30) eine Antwort auf ein Arbeitsproblem einzugeben, das eine gedankliche Arbeit der Objekterkennung für jedes der mehreren Erkennungsobjekte beinhaltet, wobei die Bewertungsvorrichtung (10) konfiguriert ist, um ein Konzentrationsverhältnis der Testperson auf der gedanklichen Arbeit unter Verwendung von mindestens einem von einer Antwortrate und einer Antwortzeit von einer Zeit, wenn das Erkennungsobjekt auf der Präsentationsvorrichtung (20) präsentiert wird, bis zu einer Zeit, wenn die Antwort in die Eingabevorrichtung (30) eingegeben wird, für jedes der mehreren Erkennungsobjekte zu bewerten;
wobei der Computer, der als Auswertungsvorrichtung (10) fungiert, umfasst:
einen Erkennungsobjektspeicherteil (121), der konfiguriert ist, um die mehreren Erkennungsobjekte zu speichern,
einen Präsentationssteuerteil (111), der konfiguriert ist, um eine Funktion des Veranlassens der Präsentationsvorrichtung (20), die mehreren Erkennungsobjekte zu präsentieren, die in dem Erkennungsobjektspeicherteil (121) gespeichert sind; und eine Funktion des Veranlassens der Präsentationsvorrichtung (20), die mehreren Optionen (31) für jedes der mehreren Erkennungsobjekte zu präsentieren, durchzuführen,
einen Arbeitsspeicherteil (122), der konfiguriert ist, um als Arbeitsinformationen die Antwortzeit und/oder die Antwortrate für jedes der mehreren Erkennungsobjekte zu speichern, und
einen arithmetischen Bewertungsteil (110), der konfiguriert ist, um einen Bewertungswert des Konzentrationsverhältnisses unter Verwendung eines Statistikwerts der Arbeitsinformationen zu berechnen, die in dem Arbeitsspeicherteil (122) gespeichert sind, während die mehreren Erkennungsobjekte auf der Präsentationsvorrichtung (20) präsentiert werden;
wobei der arithmetische Bewertungsteil (110) umfasst:
einen Histogrammerzeugungsteil (112), der konfiguriert ist, um die Antwortzeit in mehrere Abschnitte zu klassifizieren, wobei der Histogrammerzeugungsteil konfiguriert ist, um als einen Zeitbelegungsgrad ein Verhältnis einer Summe der Antwortzeit in einem Abschnitt zu einer Summe der Antwortzeit für jeden der mehreren Abschnitte zu betrachten, wobei der Histogrammerzeugungsteil konfiguriert ist, um ein Zeitbelegungsgradhistogramm zu erzeugen, das eine Verteilung des Zeitbelegungsgrads ausdrückt;
einen Anwendungsteil (113), der konfiguriert ist, um das Zeitbelegungsgradhistogramm als eine Überlagerung einer Wahrscheinlichkeitsdichtefunktion einer ersten Lognormalverteilung, die auf einen ersten bergförmigen Teil mit einem ersten Spitzenteil angewendet wird, in dem der Zeitbelegungsgrad am größten ist, und einer Wahrscheinlichkeitsdichtefunktion einer zweiten Lognormalverteilung, die auf einen zweiten bergförmigen Teil mit einem zweiten Spitzenteil angewendet wird, in dem der Zeitbelegungsgrad am nächstgrößten ist, zu betrachten; und
einen Berechnungsteil (114), der konfiguriert ist, um als eine Merkmalsmenge einen erwarteten Wert zu extrahieren, der aus der Wahrscheinlichkeitsdichtefunktion der ersten Lognormalverteilung berechnet wird, und als eine Konzentrationszeit ein Produkt der Merkmalsmenge und einer Gesamtzahl der Antwort zu berechnen, wobei der Berechnungsteil (114) konfiguriert ist, um als den Bewertungswert ein Verhältnis der Konzentrationszeit zu einer Messperiode zu berechnen, die eine Summe der Antwortzeit ist.

## Revendications

1. Appareil de mesure de rapport de concentration, comprenant :
un dispositif de présentation (20) configuré de manière à présenter une pluralité d'objets de reconnaissance à un sujet de test ;
un dispositif de saisie (30) configuré de manière à permettre au sujet de test de saisir une réponse à un problème de travail qui inclut un travail mental de reconnaissance d'objets, pour chaque objet de la pluralité d'objets de reconnaissance ; et
un dispositif d'évaluation (10) configuré de manière à évaluer un rapport de concentration du sujet de test sur le travail mental, en utilisant au moins l'un des éléments parmi un taux de réponse et un temps de réponse entre un instant où l'objet de reconnaissance est présenté sur le dispositif de présentation (20) et l'instant où la réponse est saisie dans le dispositif de saisie (30), pour chaque objet de la pluralité d'objets de reconnaissance ;
dans lequel le problème de travail comprend :
un travail consistant à extraire deux types d'éléments cognitifs prescrits ou plus inclus dans l'objet de reconnaissance, pour chaque objet de la pluralité d'objets de reconnaissance ; et
un travail consistant à choisir, parmi une pluralité d'options (31), une option adaptée aux deux éléments cognitifs prescrits ou plus extraits de l'objet de reconnaissance, pour chaque objet de la pluralité d'objets de reconnaissance, dans lequel un nombre de la pluralité d'options (31) correspond à un nombre de combinaisons de sélections définies pour chacun des deux types d'éléments cognitifs prescrits ou plus ; et
dans lequel le dispositif d'évaluation (10) comprend :
une partie de stockage d'objets de reconnaissance (121) configurée de manière à stocker la pluralité d'objets de reconnaissance ;
une partie de commande de présentation (111) configurée de manière à mettre en oeuvre une fonction consistant à amener le dispositif de présentation (20) à présenter la pluralité d'objets de reconnaissance stockés dans la partie de stockage d'objets de reconnaissance (121), et une fonction consistant à amener le dispositif de présentation (20) à présenter la pluralité d'options (31) pour chaque objet de la pluralité d'objets de reconnaissance ;
une partie de mémoire de travail (122) configurée de manière à stocker, en tant que des informations de travail, ledit au moins un élément parmi le temps de réponse et le taux de réponse pour chaque objet de la pluralité d'objets de reconnaissance ; et
une partie arithmétique d'évaluation (110) configurée de manière à calculer une valeur d'évaluation du rapport de concentration à l'aide d'une valeur statistique des informations de travail stockées dans la partie de mémoire de travail (122) tandis que la pluralité d'objets de reconnaissance est présentée sur le dispositif de présentation (20) ;
dans lequel la partie arithmétique d'évaluation (110) comprend :
une partie de génération d'histogramme (112) configurée de manière à classer le temps de réponse en une pluralité de sections, la partie de génération d'histogramme (112) étant configurée de manière à considérer, en tant qu'un degré d'occupation de temps, un rapport entre un total du temps de réponse dans une section et un total du temps de réponse pour chacune de la pluralité de sections, la partie de génération d'histogramme (112) étant configurée de manière à générer un histogramme de degré d'occupation de temps exprimant une distribution du degré d'occupation de temps ;
une partie d'application (113) configurée de manière à approximer l'histogramme de degré d'occupation de temps en tant qu'une superposition d'une fonction de densité de probabilité d'une première distribution log-normale, laquelle est appliquée à une première partie en forme de montagne présentant une première partie de crête dans laquelle le degré d'occupation de temps est le plus élevé, et une fonction de densité de probabilité d'une deuxième distribution log-normale, laquelle est appliquée à une deuxième partie en forme de montagne présentant une deuxième partie de crête dans laquelle le degré d'occupation de temps est le degré le plus élevé suivant ; et
une partie de calcul (114) configurée de manière à extraire, en tant qu'une quantité de caractéristiques, une valeur attendue calculée à partir de la fonction de densité de probabilité de la première distribution log-normale, et à calculer, en tant qu'un temps de concentration, un produit de la quantité de caractéristiques et d'un nombre total de réponses, la partie de calcul (114) étant configurée de manière à calculer, en tant que la valeur d'évaluation du rapport de concentration, un rapport entre le temps de concentration et une période de mesure qui représente un total du temps de réponse.

2. Appareil de mesure de rapport de concentration selon la revendication 1, dans lequel la partie de calcul (114) est configurée de manière à calculer, en tant qu'un temps de non-concentration au cours de la période de mesure, une valeur obtenue en soustrayant le temps de concentration de la période de mesure.

3. Appareil de mesure de rapport de concentration selon la revendication 1 ou 2,
dans lequel chaque objet de la pluralité d'objets de reconnaissance est un nombre à trois chiffres ou plus ;
dans lequel chacun des deux types d'éléments cognitifs prescrits ou plus est un caractère numérique de chaque chiffre du nombre ;
dans lequel les sélections sont des ensembles de nombres incluant deux caractères numériques ou plus, les ensembles de nombres étant divisés en trois types de groupes ou plus ; et
dans lequel le travail est un travail consistant à classer chacun des deux types d'éléments cognitifs prescrits ou plus dans un ensemble de nombres auquel appartient chacun des deux types d'éléments cognitifs prescrits ou plus.

4. Appareil de mesure de rapport de concentration selon la revendication 1 ou 2, dans lequel chaque objet de la pluralité d'objets de reconnaissance est un mot présenté de sorte que trois types d'éléments cognitifs ou plus peuvent être extraits en tant que les deux types d'éléments cognitifs prescrits ou plus.

5. Appareil de mesure de rapport de concentration selon l'une quelconque des revendications 1 à 4, comprenant en outre :
un capteur d'environnement (14) configuré de manière à mesurer au moins l'un parmi deux types d'éléments environnementaux ou plus influençant le rapport de concentration dans un environnement du sujet de test ; et
une partie de stockage de résultat (123) configurée de manière à stocker un élément environnemental mesuré par le capteur d'environnement (14) de manière à être associé à la valeur d'évaluation calculée par la partie arithmétique d'évaluation (110).

6. Appareil de mesure de rapport de concentration selon l'une quelconque des revendications 1 à 5, dans lequel le dispositif de présentation (20) et le dispositif de saisie (30) sont intégralement pourvus du dispositif d'évaluation (10).

7. Programme destiné à faire fonctionner un ordinateur en tant qu'un dispositif d'évaluation (10) configuré de manière à amener un dispositif de présentation (20) à présenter une pluralité d'objets de reconnaissance, et à considérer, en tant qu'un problème de travail, un travail consistant à extraire de deux types d'éléments cognitifs prescrits ou plus inclus dans un objet de reconnaissance pour chaque objet de la pluralité d'objets de reconnaissance ; et
un travail consistant à choisir, parmi une pluralité d'options (31), une option adaptée aux deux éléments cognitifs prescrits ou plus extraits de l'objet de reconnaissance, pour chaque objet de la pluralité d'objets de reconnaissance, dans lequel un nombre de la pluralité d'options (31) correspond à un nombre de combinaisons de sélections définies pour chacun des deux types d'éléments cognitifs prescrits ou plus, le dispositif d'évaluation (10) étant configuré de manière à permettre à un sujet de test de saisir, à partir d'un dispositif de saisie (30), une réponse à un problème de travail qui inclut un travail mental de reconnaissance d'objet pour chaque objet de la pluralité d'objets de reconnaissance, le dispositif d'évaluation (10) étant configuré de manière à évaluer un rapport de concentration du sujet de test sur le travail mental, en utilisant au moins un élément parmi un taux de réponse et un temps de réponse entre un instant où l'objet de reconnaissance est présenté sur le dispositif de présentation (20) et un instant où la réponse est saisie dans le dispositif de saisie (30), pour chaque objet de la pluralité d'objets de reconnaissance ;
dans lequel l'ordinateur fonctionnant en tant que le dispositif d'évaluation (10) comprend :
une partie de stockage d'objets de reconnaissance (121) configurée de manière à stocker la pluralité d'objets de reconnaissance ;
une partie de commande de présentation (111) configurée de manière à mettre en oeuvre une fonction consistant à amener le dispositif de présentation (20) à présenter la pluralité d'objets de reconnaissance stockés dans la partie de stockage d'objets de reconnaissance (121), et une fonction consistant à amener le dispositif de présentation (20) à présenter la pluralité d'options (31) pour chaque objet de la pluralité d'objets de reconnaissance ;
une partie de mémoire de travail (122) configurée de manière à stocker, en tant que des informations de travail, ledit au moins un élément parmi le temps de réponse et le taux de réponse pour chaque objet de la pluralité d'objets de reconnaissance ; et
une partie arithmétique d'évaluation (110) configurée de manière à calculer une valeur d'évaluation du rapport de concentration à l'aide d'une valeur statistique des informations de travail stockées dans la partie de mémoire de travail (122) tandis que la pluralité d'objets de reconnaissance est présentée sur le dispositif de présentation (20) ;
dans laquelle la partie arithmétique d'évaluation (110) comprend :
une partie de génération d'histogramme (112) configurée de manière à classer le temps de réponse en une pluralité de sections, la partie de génération d'histogramme étant configurée de manière à considérer, en tant qu'un degré d'occupation de temps, un rapport entre un total du temps de réponse dans une section et un total du temps de réponse pour chacune de la pluralité de sections, la partie de génération d'histogramme étant configurée de manière à générer un histogramme de degré d'occupation de temps exprimant une distribution du degré d'occupation de temps ;
une partie d'application (113) configurée de manière à considérer l'histogramme de degré d'occupation de temps en tant qu'une superposition d'une fonction de densité de probabilité d'une première distribution log-normale, laquelle est appliquée à une première partie en forme de montagne présentant une première partie de crête dans laquelle le degré d'occupation de temps est le plus élevé, et une fonction de densité de probabilité d'une deuxième distribution log-normale, laquelle est appliquée à une deuxième partie en forme de montagne présentant une deuxième partie de crête dans laquelle le degré d'occupation de temps est le degré le plus élevé suivant ; et
une partie de calcul (114) configurée de manière à extraire, en tant qu'une quantité de caractéristiques, une valeur attendue calculée à partir de la fonction de densité de probabilité de la première distribution log-normale, et à calculer, en tant qu'un temps de concentration, un produit de la quantité de caractéristiques et d'un nombre total de réponses, la partie de calcul (114) étant configurée de manière à calculer, en tant que la valeur d'évaluation du rapport de concentration, un rapport entre le temps de concentration et une période de mesure qui représente un total du temps de réponse.
